Europäisches Patentamt

(19) European Patent Office

Office européen des brevets

(11) Numéro de publication: **0 222 673**
**B1**

(12) **FASCICULE DE BREVET EUROPEEN**

(45) Date de publication du fascicule du brevet:
21.03.90

(51) Int. Cl.⁴: **C07C 53/18**, C07C 51/41

(21) Numéro de dépôt: 86420256.9

(22) Date de dépôt: **16.10.86**

(54) **Procédé de préparation de sels alcalins de l'acide trifluoroacétique à l'état anhydre et cristallisé.**

(30) Priorité: **18.10.85 FR 8515462**

(43) Date de publication de la demande:
**20.05.87 Bulletin 87/21**

(45) Mention de la délivrance du brevet:
**21.03.90 Bulletin 90/12**

(84) Etats contractants désignés:
**AT BE CH DE ES FR GB GR IT LI LU NL SE**

(56) Documents cités:
**GB-A- 891 858**

(73) Titulaire: **RHONE-POULENC CHIMIE, 25, quai Paul Doumer, F-92408 Courbevoie Cédex(FR)**

(72) Inventeur: **Amiet, Louis, 10, quai Saint-Vincent, F-69001 Lyon(FR)**

(74) Mandataire: **Le Pennec, Magali et al, RHONE-POULENC INTERSERVICES Service Brevets Chimie 25, Quai Paul Doumer, F-92408 Courbevoie Cédex(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation de cristaux de trifluoracétates alcalins. Elle concerne plus particulièrement un procédé de préparation de cristaux de trifluoroacétates alcalins anhydres.

On entend par sel anhydre de l'acide trifluoracétique, des sels contenant moins de 0,1 % d'eau.

Il est connu d'après le Journal of American Chemical Society 76, 4285-7 (1954) de préparer du trifluoracétate de sodium par neutralisation à l'aide de carbonate de sodium d'une solution aqueuse d'acide trifluoracétique, suivie d'une filtration, d'une évaporation à sec et d'un séchage sous vide à environ 100°C. Mais il est également connu depuis déjà très longtemps (Bulletin de la Cl. Sci.-Académie Royale de Belgique, Vol. 8, 343-370 (1922)) que "le trifluoroacétate de sodium ne cristallise de sa solution aqueuse que lorsque l'évaporation sous exsiccateur est poussée jusqu'à siccité à peu près complète". L'auteur de cet article dit ensuite que cette siccité n'est pratiquement pas possible.

En effet, d'après les essais réalisés et au vu de l'extrême hydroscopicité des sels alcalins de l'acide trifluoracétique, il est industriellement impossible d'obtenir des cristaux de sels alcalins d'acide trifluoracétique à partir de leur solution aqueuse par simple évaporation. Car, lorsqu'une solution aqueuse de trifluoroacétate alcalin est soumise à l'évaporation, on obtient un liquide extrêmement visqueux au sein duquel la cristallisation devient pratiquement impossible même lors de l'adjonction de cristaux pour amorcer la cristallisation.

La demanderesse a également essayé une méthode qui consiste à écouler une solution aqueuse de sels alcalins d'acide trifluoracétique dans un solvant organique tel que le toluène, chauffé et agité et à éliminer l'eau par distillation azéotropique. A petite échelle, cette technique est utilisable mais à l'échelle industrielle, elle est impossible à mettre en oeuvre. En effet, la phase aqueuse introduite a vite tendance à s'agglomérer pour former une deuxième phase liquide distincte.

Même en portant la phase organique à une température voisine de son point d'ébullition, de façon à éliminer le maximum d'eau, on ne peut diminuer suffisamment la teneur en eau et éviter ce démixage de la phase aqueuse.

Cette phase aqueuse a alors tendance à prendre en masse lors de sa deshydratation avec formation de blocs qui sont difficiles à extraire de l'appareillage industriel. En effet, pour extraire ceux-ci, il serait nécessaire de faire fondre la masse reccueillie mais la température de fusion des trifluoracétates alcalins (205°C par exemple pour le sel de sodium) est trop proche de celle de leur décomposition (208°C par exemple pour le sel de sodium) pour pouvoir envisager de procéder de cette manière.

Ainsi, aucun des procédés de l'art antérieur ne permet d'obtenir industriellement des sels alcalins de l'acide trifluoracétique à l'état anhydre et cristallisé.

La présente invention a permis d'atteindre cet objectif et a pour objet un procédé de préparation de sels alcalins de l'acide trifluoroacétique à l'état anhydre et cristallisé, caractérisé en ce que dans une première étape, on neutralise dans un milieu constitué essentiellement d'un alcool contenant 3 à 4 atomes de carbone, de l'acide trifluoroacétique par un agent alcalin, dans une deuxième étape, on élimine l'eau engendrée par la réaction de neutralisation, par distillation azéotropique, dans une troisième étape, on sépare les cristaux de trifluoroacétate alcalin anhydre par addition d'un hydrocarbure ne solubilisant pas le trifluoroacétate alcalin et formant un azéotrope avec l'alcool.

Parmi les hydrocarbures ne solubilisant pas le trifluoroacétate et formant un azéotrope avec l'alcool, on peut citer le cyclohexane, le benzène, le toluène ; on préfère utiliser plus particulièrement le toluène.

Parmi les alcools utilisés comme solvants, on met en oeuvre les propanols ou les butanols et de préférence les butanols car, d'une part, ils forment des azéotropes avec l'eau présentant des points d'ébullition suffisamment éloignés des alcools purs ce qui facilite leur séparation ; ils forment d'autre part, avec les hydrocarbures des azéotropes dont le point d'ébullition est lui aussi suffisamment éloigné de l'hydrocarbure pur. Comme avantage supplémentaire, ces azéotropes ainsi que les alcools purs présentent un point d'ébullition suffisamment éloigné du point de fusion des trifluoroacétates alcalins pour éviter la décomposition de ces derniers.

L'agent de neutralisation alcalin utilisé est choisi parmi les hydroxydes, les carbonates ou les hydrogénocarbonates alcalins. On préfère utiliser les carbonates car ils forment moins d'eau lors de la neutralisation de l'acide trifluoroacétique.

Après la neutralisation, l'eau est éliminée par distillation azéotropique de préférence avec l'alcool comme indiqué ci-dessus.

On ajoute ensuite de préférence de toluène qui, contrairement aux essais précédents en milieu aqueux, provoque la précipitation du trifluoroacétate alcalin.

Pour éviter des pertes de sels qui resteraient dissous dans le mélange alcool-hydrocarbure, on préfère distiller dans un premier stade de mise en oeuvre de la troisième étape l'azéotrope alcool-hydrocarbure, de façon à éliminer tout l'alcool. Au cours de cette distillation avec éventuellement une addition complémentaire d'hydrocarbure, la cristallisation se produit et les cristaux sont récupérés par simple filtration sous atmosphère sèche puis portés à l'étuve à environ 100-120°C.

Les constituants des azéotropes alcool/eau et alcool/hydrocarbure peuvent être séparés de manière connue.

Lors de la première étape, on utilise une quantité d'agent de neutralisation alcalin environ stoechiométrique par rapport à la quantité d'acide trifluoroacétique à neutraliser.

Pour une meilleure mise en oeuvre du procédé selon l'invention, lorsque l'agent de neutralisation est un carbonate, on préfère utiliser un léger excès, par rapport à la stoechiométrie, d'acide trifluoroacétique, (ceci afin d'éviter que des cristaux de trifluoroacétate alcalin entourent du carbonate inaltéré) puis lorsque tout est dissous, terminer la neutralisation à l'aide d'hydroxyde alcalin en solution aqueuse concentrée.

On utilise de préférence un rapport molaire du carbonate alcalin à l'acide trifluoracétique compris entre 0,475 et 0,5.

On utilise de préférence un rapport pondéral acide trifluoracétique à l'alcool compris entre 0,4 et 5.

Une mise en oeuvre particulièrement intéressante du procédé consiste à utiliser comme milieu de neutralisation l'azéotrope constitué d'environ 55 % en poids de butanol-2 et de 45 % en poids de toluène.

L'eau engendrée par la réaction de neutralisation est ensuite éliminée sous forme d'un ternaire eau/butanol-2/toluène qui après condensation se sépare en deux couches. Après élimination de l'eau on ajoute dans le milieu réactionnel du toluène en quantité suffisante pour permettre l'élimination du butanol-2 sous forme d'azéotrope butanol-2/toluène et pour former le milieu de précipitation du trifluoroacétate alcalin. L'azéotrope toluène-butanol-2 peut être réutilisé pour une nouvelle première étape de neutralisation.

Les trifluoroacétates alcalins sont utilisés comme agent de perfluoroalkylation pour la fabrication d'intermédiaires organiques dans l'industrie pharmaceutique et phytosanitaire (Brevet J 57139025).

L'invention sera plus complètement décrite à l'aide des exemples suivants qui ne doivent pas être considérés comme limitatifs de l'invention.

## EXEMPLE 1A

### Emploi de butanol-1 - Préparation de CF₃COONa

Dans un ballon en verre de 1 litre surmonté d'une colonne à distiller en verre, équipé d'une agitation, d'un thermomètre et d'une ampoule de coulée, on introduit 250 g de butanol-1, 53 g de Na₂CO₃ pur. Sous vive agitation, on introduit en 1 heure 115 grammes correspondant à 1 mole d'acide trifluoroacétique de pureté supérieure à 99 %. La température dans le ballon monte naturellement à environ 40°C. On maintient l'agitation 15 minutes après la fin d'addition et observe la disparition totale des cristaux de carbonate. On chauffe le ballon et distille sous pression atmosphérique à la température de 92°C en tête de colonne un mélange qui décante en deux phases après condensation.

On poursuit la distillation : la température en tête atteint 116,5-117°C ; on stoppe lorsque le volume restant est d'environ 100 cm³. On refroidit à température ambiante et n'observe aucune cristallisation. On additionne alors lentement, sous légère agitation, environ 200 cm³ de toluène, et observe la formation d'un précipité cristallin blanc. On filtre, rince le précipité par environ 25 cm³ de toluène et sèche à l'étuve à 120°C. On obtient ainsi 105 g (0,77 mole) de cristaux secs de CF₃COONa (rendement = 77 %).

## EXEMPLE 1B

Même appareillage que pour l'exemple 1A

Dans le ballon, on charge 200 grammes de butanol-1 et 106 grammes de Na₂CO₃ pur. Sous agitation, on écoule, en 30 minutes environ, 235 grammes d'acide trifluoroacétique (pureté supérieure à 99 %).

La température dans le ballon s'élève depuis 25°C jusqu'à 55°C.

On chauffe ensuite et observe à 90°C une solution homogène. Sur un prélèvement, on constate que le pH est de 1 et le ramène à 6 par un ajout goutte à goutte d'une solution aqueuse de NaOH à environ 50% ; 3 g de cette solution sont nécessaires pour cela. On chauffe à reflux et distille à 89-90°C en tête de colonne, un mélange qui après condensation se sépare en deux couches (la distillation est arrêtée dès l'amorce d'une montée en température au-delà de 90°C) : une couche dense de 13 grammes, une légère de 22 g. A la fin de cette distillation, on observe l'amorce d'une précipitation. La température dans le ballon, de 119°C au début de la distillation et de 129 à la fin, est ramenée alors à 105-110°C, puis on ajoute alors, toujours sous agitation, 300 grammes de toluène. On distille alors un mélange, passant au début à une température de 100°C en tête et évoluant lentement vers 105°C, pesant 484 g, cependant qu'on alimente en continu du toluène, puis une deuxième fraction passant à 105°C et pesant 348 g ; la masse de toluène ajoutée au cours de la distillation est de 600 g. Puis le température en tête monte assez vite à 109°C cependant qu'on distille encore 45 g de toluène. La distillation est alors arrêtée, le ballon refroidi à environ 30°C et son contenu passé sur filtre. Le poids de précipité imprégné de toluène est de 295 grammes, et, après avoir été porté à l'étuve à 115°C, 270 grammes (rendement d'environ 97 %).

## EXEMPLE 2

### Emploi d'isobutanol (ou méthyl-2, propanol-1)

On utilise le même montage que dans l'exemple 1.

On introduit dans le ballon 106 g de Na₂CO₃ pur et 200 g d'isobutanol, puis en 30 minutes, sous agitation, 230,3 g (2 moles) d'acide trifluoroacétique (TFA). On chauffe le mélange à reflux et observe que la dissolution est totale après une heure. On distille alors, à 87,5-89°C en tête de colonne, un mélange qui décante en deux couches, puis de 89 à 108°C, une fraction intermédiaire de 22g. On ajoute progressivement, par l'ampoule de coulée, 412 g de toluène à une vitesse telle que le niveau dans le ballon reste sensiblement constant au cours de la poursuite de la distillation. Un précipité blanc se forme progressivement. On distille ainsi à 98-99,5°C en tête de colonne une fraction pesant 314 g puis de 99,5 à 110°C une fraction de 21 g, enfin 50 g à cette dernière température de 110°C. Le ballon est refroidi à température ambiante, puis son contenu filtré sur un filtre de verre fritté. Le précipité recueilli, imprégné de toluène, pèse 293 g et, après avoir été porté 3 heures à l'étuve à 115°C, 267 g ce qui correspond à un rendement de 97,4 % en trifluoroacétate de sodium anhydre. Il se présente sous forme de cristaux blancs, fins. L'analyse thermique met en évidence une fusion vers 208°C suivie d'une décomposition vers 212°C. La teneur en eau déterminée par la méthode de K. Fisher sur le produit séché indique une valeur de 400 mg/kg.

EXEMPLE 3

On effectue une nouvelle préparation de CF₃COONa comme précédemment mais dans un ballon de 6 litres.

On met en oeuvre 848 g (8 moles) de Na₂CO₃ pur et 1.600 g d'isobutanol. On introduit en 1 heure 30 mn, dans la suspension agitée, 1.892,4 g (16,45 moles) de CF₃COOH. On laisse CO₂ se dégager librement.

On porte la température à 85°C après 15 mn, on observe que tout le milieu réactionnel est devenu limpide. On additionne progressivement une solution aqueuse à environ 50 % de NaOH pure jusqu'à obtention d'un pH de 5 à 6, ce qui nécessite 42 g de solution alcaline.

On procède alors à la distillation de l'azéotrope isobutanol-H₂O, la température en tête de colonne se situant entre 86 et 89°C, celle dans le ballon passant de 108 à 118°C. Le distillat décante en deux couches :
- 1 phase aqueuse dense, pesant 102 g, à environ 90 % d'eau
- 1 phase supérieure de 415 g à 10 % d'eau environ.

On poursuit la distillation et sépare une nouvelle fraction passant entre 98 et 100°C, pesant 220 g, à 4,9 % d'eau.

On refroidit le ballon à 95°C et ajoute 950 g de toluène en maintenant cette température. On constate que le milieu est encore homogène. On ajoute alors progressivement, sous lente agitation, 920 g de toluène et observe le début de la cristallisation dès la reprise de l'addition de toluène. La distillation est ensuite reprise tout en maintenant l'agitation et en alimentant simultanément du toluène. On sépare ainsi :
- une petite fraction de 41 g passant de 76 à 90°C laissant décanter 2 g de phase aqueuse
- ensuite une fraction de 2.262 g passant à 99-100°C contenant 0,4 % d'eau, 43,2 % d'isobutanol et 56,4 % de toluène.

La charge supplémentaire de toluène est de 1.000 g, additionnée durant la distillation de ces deux fractions. On stoppe alors l'alimentation de toluène et poursuit la distillation.

La température de tête monte à 109°C et on sépare une dernière fraction de 385 g, constituée par du toluène et contenant 220 ppm d'eau.

Le ballon est refroidi. On filtre et sèche à l'étuve le précipité comme précédemment à 115°C. La masse de CF₃COONa recueillie, à 480 ppm d'eau d'après l'analyse, est de 2.198 g (16,16 moles). On obtient un rendement de trifluoroacétate de sodium d'environ 98 %.

EXEMPLE 4

Préparation de CF₃COO Li solide - Emploi d'isobutanol

On utilise l'appareillage de l'exemple N° 1. On introduit dans le ballon 74 g de Li₂CO₃ pur et 200 g d'isobutanol, puis on ajoute par l'ampoule, en 1 heure, 234,8 g de CF₃COOH (2,04 moles) au contenu du ballon agité. On chauffe ensuite le ballon jusqu'à 80°C. Le pH du milieu est sensiblement 1. On ajoute alors une solution aqueuse saturée de lithine pure, jusqu'à ce que le pH atteigne 5. On distille alors à 88-89°C en tête un liquide qui décante en deux phases, la phase inférieure riche en eau pesant 21 g et la phase supérieure 74 g. On sépare ensuite une fraction de 25 g de 89 à 106°C en tête de colonne, la température dans le ballon atteignant 138°C. On laisse refroidir à 95°C puis introduit 400 g de toluène et reprend la distillation. On sépare ainsi à 99-100°C une fraction de 328 g tout en introduisant 300 g de toluène dans le ballon, puis on distille encore 50 g, jusqu'à atteindre 108°C en tête. On laisse refroidir le ballon jusqu'à 50°C, filtre et sèche à 115°C le solide obtenu. On obtient 246 g de produit à 0,08 % d'eau d'après l'analyse. Le rendement en trifluoroacétate de lithium anhydre cristallisé obtenu est d'environ 99,5 %.

EXEMPLE 5

Préparation de CF₃COOK

Dans le même appareillage que précédemment, on introduit 138 g de K₂CO₃ pur, 200 g d'isobutanol et, suivant le même processus que précédemment, 235 g (2,04 moles) de CF₃COOH. Le pH d'environ 1 à la fin de la réaction est ramené à 6 par addition d'environ 6 g de KOH à environ 50 %. On suit le même processus de séparation de l'azéotrope isobutanol-H₂O. On sépare ainsi successivement une fraction de 61 g à 88-89°C qui décante en deux phases, puis une fraction intermédiaire de 44 g jusqu'à 106°C.

Après avoir ramené le ballon à 95°C, on ajoute 400 g de toluène, distille 330 g à 99-100°C de mélange azéotropique toluène/isobutanol tout en ajoutant de façon continue encore 150 g de toluène, sépare encore une fraction de 50 g jusqu'à 110°C en tête. Après refroidissement à environ 50°C, filtration et séchage, on récupère 301 g de produit contenant 550 ppm d'eau (0,055 %). Le rendement en trifluoroacétate de potassium est d'environ 96 %.

EXEMPLE 6

Emploi de butanol-2 - Préparation de CF₃COONa

On met en oeuvre, dans le même appareillage, 106 g de Na₂CO₃ pur, 200 g de butanol-2, puis selon le processus déjà décrit, 235 g (2,04 moles) de CF₃COOH et une heure. Le pH final d'environ 1 est ramené à 5 par addition de 4 g de solution aqueuse de NaOH concentrée.

On distille, tout en maintenant une légère agitation dans le ballon :

- à 84-85°C, une fraction homogène après condensation de 64 g
- puis jusqu'à 93°C une fraction de 16 g.

On observe alors le début d'une cristallisation dans le liquide du ballon. Après refroidissement du ballon, on introduit 300 g de toluène et reprend la distillation. On sépare successivement :
- une fraction de 13 g jusqu'à 93°C en tête de colonne
- " " de 221 g à 93-94°C, que l'analyse indique à 54,3 % de butanol-2
- une fraction de 9 g jusqu'à 108°C
- enfin, une fraction de 40 g à 110°C.

On refroidit à 40°C, filtre sur verre fritté, sèche à 115°C et obtient 275 g de produit final qui correspond à un rendement en trifluoroacétate de sodium de 99 %.

EXEMPLE 7

Dans le même appareillage, on introduit cette fois un mélange de 200 g de butanol-2 avec 160 g de toluène, correspondant sensiblement à l'azéotrope toluène/butanol-2, et 106 g de $Na_2CO_3$ pur. On procède ensuite comme précédemment à l'addition de 235 g de $CF_3COOH$ à plus de 99 % soit 2,04 moles de $CF_3COOH$ par heure, ce qui porte la température à 50°C. On chauffe ensuite à 70°C et constate que tout est dissous. Le pH est ramené à 5/6 par NaOH 50 % puis on procède à la distillation.

On sépare successivement :
- à 77-78°C en tête de colonne, une fraction qui se sépare en deux phases, une phase inférieure de 19 g dosée à 91 % d'eau et une phase organique de 51 g contenant, d'après l'analyse, 37 % de butanol-2, 56,2 % de toluène et 3,8 % d'eau.
- à 93-94°C en tête, en ajoutant en continu du toluène dans le ballon pour compenser le volume distillé (ce qui provoque rapidement la cristallisation de $CF_3COONa$) un distillat de 294 g contenant d'après l'analyse, 58,5 % de butanol-2, 40,9 % de toluène et 0,28 % d'eau.
- de 95 à 109°C, une fraction de 10 g
- enfin 50 g à 109-110°C.

Le contenu du ballon est, comme dans les exemples précédents, filtré et le solide séché à 115°C. On recueille finalement 274 g de produit à 0,08 % d'eau ce qui correspond à un rendement de 97,9 % en trifluoroacétate de sodium.

EXEMPLE 8

Utilisation d'isopropanol

Dans le même appareillage, on introduit 200 g d'isopropanol, 106 g de $Na_2CO_3$ et fait réagir 235 g (2,04 moles) de $CF_3COOH$. Quand le dégagement de $CO_2$ a cessé, la solution est à 50°C et il ne subsiste plus de solide. On ramène le pH à 6 par NaOH puis distille, après avoir rajouté 50 g d'isopropanol supplémentaire :
- à 76°C en tête, une fraction de 170 g
après refroidissement à 90°C, ajout de 350 g de toluène (ce qui provoque le début de la précipitation) et reprise du chauffage,

- une fraction de 168 g passant à 76-77°C et montant rapidement à 100°C.
- enfin de 100 à 109°C, 50 g.

On refroidit, filtre et sèche, et obtient 275 g de trifluoroacétate de sodium ce qui correspond à un rendement de 99,3 %.

EXEMPLE 9

Emploi de butanol-2, de cyclohexane à la place de toluène.

Préparation de trifluoroacétate de sodium.

Dans le même appareillage que précédemment, on introduit 106 g de $Na_2CO_3$ pur, 200 g de butanol-2, puis selon le processus déjà décrit, 235 g d'acide trifluoroacétique (à plus de 99 % de $CF_3COOH$) en 1 heure environ. Comme précédemment, le pH est ramené à environ 5 par quelques grammes de NaOH concentré.

On sépare par distillation :
- à 85-86°C en tête de colonne, une fraction de 84 grammes se séparant en une couche dense de 11 g et une couche légère de 73 g
- entre 86 et 100°C, une fraction de 4 g.

La distillation est alors arrêtée, le contenu du ballon refroidi à 75°C. On ajoute par l'ampoule de coulée 300 g de cyclohexane, sous agitation. On observe alors la séparation du liquide en 2 couches et un début de précipitation de solide blanc fin ($CF_3COONa$).

On reprend la distillation et sépare, à 75-76°C en tête de colonne, tout en ajoutant par l'ampoule de coulée du cyclohexane de manière à maintenir un niveau liquide à peu près constant dans le bouilleur, une fraction de 735 grammes. La quantité de cyclohexane introduite simultanément est de 650 grammes.

La distillation est alors arrêtée, le contenu du ballon refroidi à environ 30°C puis passé sur un filtre en verre fritté. On recueille une masse de cristaux imprégnée de cyclohexane de 290 g, et après séchage à l'étuve à 120°C, de 268 grammes, correspondant à un rendement d'environ 96-97 %.

**Revendications**

1. Procédé de préparation de sels alcalins de l'acide trifluoroacétique à l'état anhydre et cristallisé caractérisé en ce que dans une première étape, on neutralise dans un milieu constitué essentiellement d'un alcool contenant 3 ou 4 atomes de carbone, de l'acide trifluoroacétique par un agent alcalin, dans une deuxième étape, on élimine l'eau engendrée par la réaction de neutralisation, par distillation azéotropique, dans une troisième étape, on sépare les cristaux de trifluoroacétate alcalin anhydre par addition d'hydrocarbure ne solubilisant pas le trifluoroacétate alcalin et formant un azéotrope avec l'alcool.

2. Procédé selon la revendication 1 caractérisé en ce que l'hydrocarbure est choisi parmi le cyclohexane, le benzène ou le toluène.

3. Procédé selon la revendication 2 caractérisé en ce que l'hydrocarbure est le toluène.

4. Procédé selon la revendication 1 caractérisé en ce que l'alcool est un butanol.

5. Procédé selon la revendication 1 caractérisé en ce que l'agent alcalin est choisi parmi les hydroxydes, les carbonates, et les hydrogénocarbonates alcalins.

6. Procédé selon la revendication 5 caractérisé en ce que l'agent alcalin est un carbonate alcalin et un hydroxyde alcalin.

7. Procédé selon la revendication 6 caractérisé en ce que le rapport molaire acide trifluoroacétique à l'agent alcalin est stoechiométrique.

8. Procédé selon les revendications 6 et 7 caractérisé en ce que le rapport molaire du carbonate à l'acide trifluoroacétique est compris entre 0,475 et 0,5 et la quantité d'hydroxyde alcalin utilisée permet d'atteindre la stoechiométrie.

9. Procédé selon la revendication 1 caractérisé en ce que avant la troisième étape, on procède à une élimination de l'alcool à l'aide de l'azéotrope alcool-hydrocarbure.

10. Procédé de préparation de sels alcalins de l'acide trifluoroacétique à l'état anhydre et cristallisé, caractérisé en ce que dans une première étape, on neutralise dans un milieu constitué d'un mélange toluène-alcool contenant 3 à 4 atomes de carbone, de l'acide trifluoroacétique par un agent alcalin, dans une deuxième étape, on élimine l'eau par distillation azéotropique, dans une troisième étape, on sépare les cristaux de trifluoroacétate alcalin anhydre par addition supplémentaire de toluène.

## Claims

1. Process for preparing alkali metal salts of trifluoroacetic acid in the anhydrous and crystallized state, characterized in that, in a first stage, trifluoroacetic acid is neutralized with an alkaline agent in a medium consisting essentially of an alcohol containing to 4 carbon atoms, in a second stage, the water formed by the neutralization reaction is removed by azeotropic distillation, and in a third stage, the crystals of anhydrous alkali metal trifluoroacetate are separated by adding a hydrocarbon which does not solubilize the alkali metal trifluoroacetate and which forms an azeotrope with the alcohol.

2. Process according to claim 1, characterized in that the hydrocarbon is chosen from cyclohexane, benzene or toluene.

3. Process according to claim 2, characterized in that the hydrocarbon is toluene.

4. Process according to claim 1, characterized in that the alcohol is a butanol.

5. Process according to claim 1, characterized in that the alkaline agent is chosen from alkali metal hydroxides, carbonates and hydrogen carbonates.

6. Process according to claim 5, characterized in that the alkaline agent is an alkali metal carbonate and an alkali metal hydroxide.

7. Process according to claim 6, characterized in that the mole ratio of trifluoroacetic acid to the alkaline agent is stoichiometric.

8. Process according to claims 6 and 7, characterized in that the mole ratio of the carbonate to trifluoroacetic acid is between 0.475 and 0.5, and the amount of alkali metal hydroxide used enables stoichiometric proportions to be attained.

9. Process according to claim 1, characterized in that, before the third stage, the alcohol is removed by means of the alcohol/hydrocarbon azeotrope.

10. Process for preparing alkali metal salts of trifluoroacetic acid in the anhydrous and crystallized state, characterized in that, in a first stage, trifluoroacetic acid is neutralized with an alkaline agent in a medium consisting of a mixture of toluene and an alcohol containing 3 to 4 carbon atoms, in a second stage, the water is removed by azeotropic distillation, and in a third stage, the crystals of anhydrous alkali metal trifluoroacetate are separated by a further addition of toluene.

## Patentansprüche

1. Verfahren zur Herstellung von Alkalisalzen der Trifluoressigsäure im wasserfreien und kristallinen Zustand, dadurch gekennzeichnet, daß man in einem ersten Schritt in einem im wesentlichen aus einem Alkohol mit drei oder vier Kohlenstoffatomen bestehenden Medium Trifluoressigsäure mit einem alkalischen Agens neutralisiert, in einem zweiten Schritt das durch die Neutralisationsreaktion gebildete Wasser durch azeotrope Destillation eliminiert, und man in einem dritten Schritt die Kristalle des wasserfreien Alkalitrifluoracetats durch Zugabe eines Kohlenwasserstoffs, der das Alkalitrifluoracetat nicht löst und mit dem Alkohol ein Azeotrop bildet, abtrennt.

2. Verfahren nach Anspruch 1, dadurch gekennzeichnet, daß der Kohlenwasserstoff ausgewählt wird aus Cyclohexan, Benzol oder Toluol.

3. Verfahren nach Anspruch 2, dadurch gekennzeichnet, daß der Kohlenwasserstoff Toluol ist.

4. Verfahren nach Anspruch 1, dadurch gekennzeichnet, daß der Alkohol ein Butanol ist.

5. Verfahren nach Anspruch 1, dadurch gekennzeichnet, daß das alkalische Agenz ausgewählt wird aus den Alkalihydroxiden, -carbonaten und -hydrogencarbonaten.

6. Verfahren nach Anspruch 5, dadurch gekennzeichnet, daß das alkalische Agenz ein Alkalicarbonat und ein Alkalihydroxid ist.

7. Verfahren nach Anspruch 6, dadurch gekennzeichnet, daß das molare Verhältnis Trifluoressigsäure zu alkalischem Agenz stöchiometrisch ist.

8. Verfahren nach den Ansprüchen 6 und 7, dadurch gekennzeichnet, daß das molare Verhältnis von Carbonat zu Trifluoressigsäure zwischen 0,475 und 0,5 liegt und die eingesetzte Menge an Alkalihydroxid es ermöglicht, stöchiometrische Verhältnisse zu erreichen.

9. Verfahren nach Anspruch 1, dadurch gekennzeichnet, daß man vor dem dritten Schritt eine Eliminierung des Alkohols mit Hilfe des Azeotrops Alkohol-Kohlenwasserstoff durchführt.

10. Verfahren zur Herstellung von Alkalisalzen der Trifluoressigsäure in wasserfreiem und kristallinem Zustand, dadurch gekennzeichnet, daß man in einem ersten Schritt in einem Medium bestehend aus einem Gemisch Toluol und Alkohol mit drei bis vier Kohlenstoffatomen Trifluoressigsäure mit einem alkalischen Agenz neutralisiert, in einem zweiten Schritt das Wasser durch azeotrope Destillation eliminiert und in einem dritten Schritt die Kristalle des wasserfreien Alkalitrifluoracetats durch zusätzliche Zugabe von Toluol abtrennt.